# EUROPEAN PATENT SPECIFICATION

(11) **EP 3 363 491 B1**
(45) Date of publication and mention of the grant of the patent: **11.12.2019**
(21) Application number: 16855124.0
(22) Date of filing: 14.10.2016
(51) Int. Cl.: A61M 37/00, A61B 5/15, A61M 5/46

(54) **ADMINISTRATION INSTRUMENT**
VERABREICHUNGSINSTRUMENT
INSTRUMENT D'ADMINISTRATION

(30) Priority: 15.10.2015 JP 2015203823
(43) Date of publication of application: 22.08.2018
(73) Proprietor: Toppan Printing Co., Ltd., Tokyo 110-0016 (JP)
(72) Inventor: SUMIDA, Tomoya, Tokyo 110-0016 (JP); ASAI, Ryoichi, Tokyo 110-0016 (JP)
(74) Representative: TBK
(86) International application number: PCT/JP2016/004588
(87) International publication number: WO 2017/064870

(56) References cited:
- WO-A1-2015/136639
- JP-A- 2000 037 456
- JP-A- 2004 065 775
- JP-A- 2004 065 775
- JP-A- 2007 529 247
- JP-A- 2007 529 247
- JP-A- 2012 515 604
- US-A1- 2005 137 525
- US-A1- 2007 142 885
- US-A1- 2011 237 925

## Description

### [Technical Field]

The present invention relates to administration devices having a needle-shaped body.

### [Background Art]

Injection is commonly used for administration of a drug such as vaccine into the body. Although injection is a highly safe method for administration, it often causes severe pain since an injection needle is deeply pierced into the body to deliver the drug into the subcutaneous tissue. Further, particularly in developing countries, there are many issues such as infection by reuse of injection needles and needle stick accidents.

Therefore, as an alternative drug administration method to injection, attention has been paid to use of a needle-shaped body having a plurality of projections of micron order that pierce the skin for direct administration of a drug into the skin. According to this method, it is possible to almost avoid producing pain during piercing into the skin since the length of the projections is controlled not to reach the nerve cells in the dermis layer.

Further, when vaccine is intradermally administered using a needle-shaped body, the amount of vaccine used can be reduced compared to subcutaneous injection since antigen presenting cells are abundant in the skin.

The projections of a needle-shaped body must have a thinness and sharpness sufficient for puncturing the skin and a length sufficient for intradermal drug delivery. Accordingly, the projections desirably have a diameter of several tens of micrometers to several hundreds of micrometers and a length that penetrates through the stratum corneum which is the outermost layer of the skin but does not reach the nerve plexus, which is specifically several hundreds of micrometers to several millimeters.

Materials for the needle-shaped body are required to be harmless to the human body even if the projection is broken and left in the body. For such materials, biocompatible resins such as medical grade silicone, maltose, poly lactic acid, and dextran are regarded as promising materials (see PTL 1).

### [Citation List]

### [Patent Literature]

PTL 1: JP-2005-21677 A US2011237925; WO2015136639

### [Summary of the Invention]

### [Technical Problem]

For low cost mass production of a microstructure such as the needle-shaped body by using the above resin materials, a transfer molding process such as injection molding, imprinting or casting is effective.

As methods of intradermal drug administration using a needle-shaped body, there are methods which have been proposed, including a method of applying a drug on the skin surface before or after puncturing the skin by a needle-shaped body, a method of puncturing the skin by a needle-shaped body having a projection on which a drug is applied in advance or a needle-shaped body made of a material containing a drug therein, and a method of puncturing the skin by a needle-shaped body in which a through hole is formed and then administering a drug into the skin via the through hole.

For example, when a needle-shaped body is used to administer a drug, especially a vaccine into the skin, control of the administration depth is of importance. For example, if a needle-shaped body having a projection of a length that can pierce the dermis layer of the skin of an adult is used for a child whose skin is thin, there is a risk that the projection may penetrate through the skin and pierce the hypodermis.

Further, when puncture into the dermis layer immediately under the epidermis layer is performed targeting both the Langerhans cells abundant in the epidermis layer and the dermal dendritic cells abundant in the dermis layer, which are antigen-presenting cells, puncture into the epidermis layer may fail if a needle-shaped body having a projection of a length suitable for children is used for an adult whose skin is thick.

Both cases described above may lead to an unintended immune response.

In order to address such a problem, adjustment of a puncture load, a puncture speed, or the like of the needle-shaped body can be performed depending on the thickness of the skin, for example. However, there are technical difficulties in precise adjustment. Further, when needle-shaped bodies having projections of different lengths are used depending on the thickness of the skin, a plurality of types of needle-shaped bodies should be prepared, which causes an increase in the cost of vaccine administration using a needle-shaped body.

The present invention has been made in view of the above problem, and is directed to provide an administration device that can change the puncture depth depending on the thickness of the skin when a needle-shaped body has projections of the identical length.

### [Solution to Problem]

An aspect of the present invention is an administration device including a needle-shaped body having a substrate and a projection protruding from one surface of the substrate, and a puncture depth adjustment section disposed on one surface of the substrate and having at least one film member that is detachable from the needle-shaped body.

Further, a base to which the needle-shaped body and the puncture depth adjustment section are fixed may be further provided.

Further, a height of the projection may be larger than a thickness of the puncture depth adjustment section.

Further, the film member may be an adhesive film.

Further, the film member may have a tab.

Further, the film member may be shaped as an annulus having an opening whose opening area is larger than an area of a portion on the one surface of the substrate in which the projection is formed.

### [Advantageous Effects of Invention]

The administration device according to the present invention has a simple configuration made up of the needle-shaped body having the projections, and the puncture depth adjustment section. Therefore, the manufacturing cost can be reduced. Further, the puncture depth can be adjusted by simply peeling the required number of sheets of the film members. Therefore, ease of use can be ensured.

According to the administration device of the present invention, the puncture depth can be changed depending on the thickness of the skin when a needle-shaped body has projections of the identical length.

### [Brief Description of the Drawings]

Fig. 1 is a perspective view of an administration device according to a first embodiment of the present invention.
Fig. 2 is an exploded view of the administration device according to the first embodiment of the present invention.
Fig. 3 includes a perspective view and a cross-sectional view of a first modified example of the administration device.
Fig. 4 is a perspective view of a second modified example of the administration device.
Fig. 5 is a partial cross-sectional view of the administration device according to the first embodiment of the present invention.
Fig. 6 is a cross-sectional view which illustrates a usage of the administration device.
Fig. 7 is a cross-sectional view which illustrates a usage of the administration device.
Fig. 8 includes a perspective view and a cross-sectional view of the administration device according to a second embodiment of the present invention.
Fig. 9 includes a perspective view and a cross-sectional view of a first modified example of the administration device according to the second embodiment of the present invention.
Fig. 10 is a perspective view of a second modified example of the administration device according to the second embodiment of the present invention.
Fig. 11 is a cross-sectional view of the administration device with a protective cover according to a third embodiment of the present invention.
Fig. 12 is a cross-sectional view of the administration device with a protective cover according to the third embodiment of the present invention.

### [Description of Embodiments]

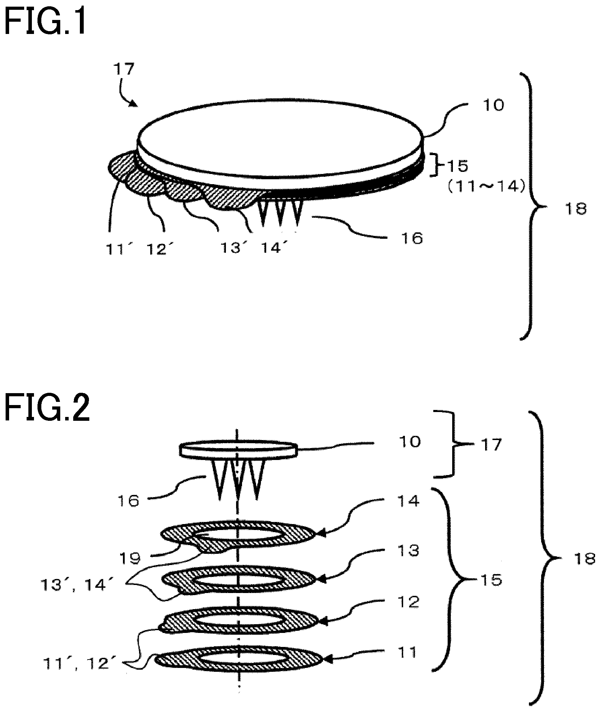

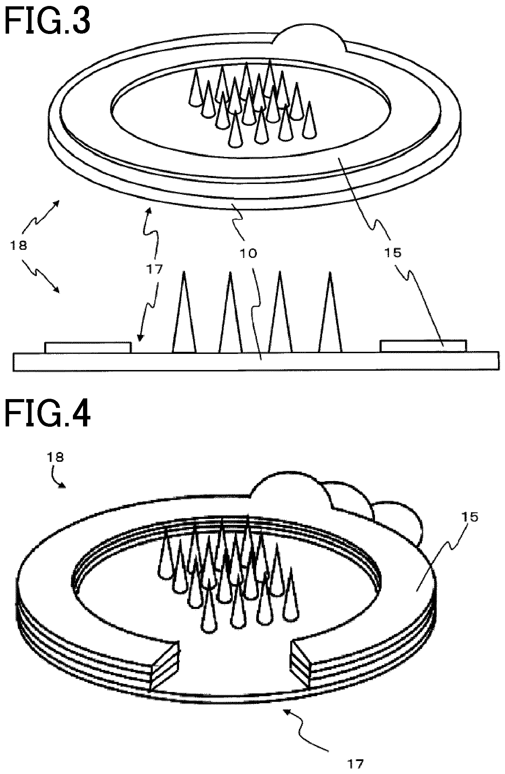

With reference to Figs. 1 to 4, an administration device (18) according to a first embodiment of the present invention will be described. Fig. 1 is a perspective view of the administration device (18), and Fig. 2 is an exploded view of the administration device (18). Further, Fig. 3 includes a perspective view and a cross-sectional view of a first modified example of the administration device (18), and Fig. 4 is a perspective view of a second modified example of the administration device (18). In the following description, the same or corresponding components in the embodiments are denoted by the same reference numbers, and the description thereof will be omitted.

An administration device of the present invention is used for puncturing the skin of humans and animals. The administration device (18) includes a substrate (10), a needle-shaped body (17) having projections (16) protruding from one surface of the substrate (10), and a puncture depth adjustment section (15) made up of one or more film members (11 to 14) provided on the one surface of the substrate (10) to be detachable from the needle-shaped body (17). In the illustrated example, the film member (14) is removably bonded to the substrate 10, and the film members (13), (12), and (11) are removably laminated in this order on the film member (14). That is, these film members (11 to 14) are each detachable from the needle-shaped body (17), which is a main body of the administration device (18). The substrate (10) is not limited to a disk shape as illustrated, and may be in any shape. Further, the projection (16) is not limited to the illustrated shape as long as it can puncture the skin. For example, the shape of the projection (16) may be selected from a conical shape and a polygonal pyramid shape such as a quadrangular pyramid shape. Further, a shape which does not have a pointed tip such as a cylinder shape, a polygonal columnar shape, or a truncated prism shape may be selected. These shapes can also be used in combination with each other. Moreover, a height of the projection (16) of the needle-shaped body (17) from a surface of the substrate (10) is larger than a thickness of the puncture depth adjustment section (15). Accordingly, as shown in Fig. 1, as viewed from the lateral side of the administration device (18) (in a horizontal direction to the drawing sheet of Fig. 1), the tip of the projection (16) protrudes from the planar surface of the puncture depth adjustment section (15).

A material for the needle-shaped body (17) is, but is not limited to, preferably a biocompatible material, which may be a metal material, thermoplastic resin, or water soluble material. Examples of the metal material include stainless steel, titanium, manganese, and the like. Examples of the thermoplastic resin include polylactic acid, cycloolefin copolymer, polyethylene, polypropylene, polycarbonate, polyglycolic acid, and the like. The water soluble material may be a water soluble polymer or polysaccharide. Examples of the water-soluble polymer include carboxymethyl cellulose (CMC), methylcellulose (MC), hydroxylpropyl cellulose (HPC), hydroxypropyl methylcellulose (HPMC), polyvinyl alcohol (PVA), polyacrylic acid polymer, polyacrylic amide (PAM), polyethylene oxide (PEO), pullulan, alginate, pectin, chitosan, and chitosan succinamide. In addition, the substrate (10) and the projection (16) may be formed of different materials. Further, the substrate (10) may have a multi-layered configuration. Similarly, the projection (16) may have a multi-layered configuration.

The needle-shaped body (17) may contain a drug therein. When the projection (16) of the needle-shaped body (17) is made of a metal or thermoplastic resin, a drug may be applied on a surface of the projection (16). Further, when the projection (16) of the needle-shaped body (17) is made of a water soluble material, a drug can be contained inside the projection (16). Moreover, the water soluble material that constitutes the projection (16) may serve as a drug.

Any kind of drug may be used as long as it works when administered into the skin. Examples of a drug include various types of proteins, pharmacologically active agents, or cosmetic compositions, which are appropriately selected depending on the purpose. Examples of a pharmacologically active agent include vaccines such as influenza vaccine, pain relievers for cancer patients, insulin, biologics, gene therapy agents, injections, oral agents, skin application preparations and the like. In transdermal administration using the needle-shaped body (17), a drug is administered into a hole created in the skin. Therefore, transdermal administration using the needle-shaped body (17) can be applied to not only administration of the pharmacologically active agents used in the conventional transdermal administration, but also administration of pharmacologically active agents that require hypodermic injection. In particular, transdermal administration using the needle-shaped body (17) is suitable for administration of an injection medication such as vaccines for children since it does not cause pain to a patient during administration. Further, transdermal administration using the needle-shaped body (17) is suitable for administration of an oral medication for children who have difficulty in swallowing an oral medication since it does not require a patient to swallow a drug in administration.

Cosmetic compositions are compositions for use as cosmetics or beauty products. Examples of a cosmetic composition include humectants, colorants, fragrance, and physiologically active agents exhibiting cosmetic effects such as improvement effect on wrinkles, acne, stretch marks or the like, and improvement effect on hair loss or the like. When an aromatic material is used as a cosmetic composition, a fragrance can be imparted to the needle-shaped body (17). Accordingly, the needle-shaped body (17) suitable for use as a beauty product can be obtained.

The needle-shaped body (17) can be produced by using various known techniques. For example, when a resin is used as a material, molding techniques such as injection molding, extrusion molding, imprinting, hot embossing, and casting can be used.

Further, when a metal or silicon is used as a material of the needle-shaped body (17), machining such as cutting or grinding, and etching techniques such as dry etching and wet etching can be used.

For example, the needle-shaped body (17) of the present invention is produced as follows. First, an intaglio plate having a recess formed in conformity with the shape of the needle-shaped body (17) is produced. In producing the intaglio plate, an original plate having the shape identical to the shape of the desired needle-shaped body (17) is produced. The shape of the original plate determines the shape of the needle-shaped body (17) to be produced. The original plate may be produced by a known method suitable for the shape of the original plate, for example, by using a micromachining technique. Micromachining technique includes lithography, wet etching, dry etching, sand blasting, laser processing, micromachining and the like.

Next, the intaglio plate having an inverted shape of recesses and projections of the original plate is produced from the original plate. The intaglio plate is formed by a known shape-transfer technique. The shape-transfer technique includes a method of producing the intaglio plate made of nickel by nickel electroforming, a method of transfer molding using a molten resin, and the like. Accordingly, the intaglio plate having recesses which are depressions shaped in conformity with the projections, is produced.

Then, the recesses of the produced intaglio plate are filled with a material for forming the needle-shaped body. When the needle-shaped body (17) is made of a thermoplastic resin, a molded product (needle-shaped body (17)) is produced by thermal pressing of a sheet shaped thermoplastic resin placed on the recesses 51 so as to fill the thermoplastic resin into the recesses or by injection molding of a thermoplastic resin into the mold having the intaglio plate so as to fill the thermoplastic resin into the recesses.

On the other hand, when the needle-shaped body (17) is made of a water soluble material, a material solution containing a water soluble material and a drug is prepared. The flowability of the material solution is preferably adjusted to an extent such that the material solution is smoothly filled into the recesses by adjusting the amount of the solvent or the like as appropriate. The way of supplying the material solution into the recesses may be appropriately selected from known methods taking into consideration the shape, size, or the like of the recesses. The material solution can be supplied by methods such as spin coating, use of dispenser, casting, and ink jetting. The material solution may be supplied to the recesses under normal pressure, but preferably under reduced pressure or vacuum in order to smoothly supply the material solution into the recesses. The amount of material solution supplied to the recesses is preferably at least such an extent that the depression of the shape in conformity with the inner projection is entirely covered with the material solution. As the material solution filled into the recesses is dried and solidified, the needle-shaped body (17) is produced.

For the film members (11 to 14) that constitute the puncture depth adjustment section (15), a single layered film or a multi-layered film can be used. Among others, an adhesive film can be advantageously used.

An exemplary configuration of the adhesive film for use as the film members (11 to 14) may include an adhesive layer on the substrate sheet. The substrate sheet may be formed of, for example, a resin film made of polyolefin resin such as polyethylene, polypropylene, and cyclic polyolefin, polyester resin such as nylon and polyethylene terephthalate, polyvinyl chloride, polyvinylidene chloride, or polystyrene, polyurethane, polyvinyl alcohol, polyimide or the like. Further, the adhesive layer may be formed of, for example, a silicone, urethane, epoxy, or acrylic adhesive. However, the adhesive sheet is not limited to these materials. Further, the adhesive film may be provided with a peeling layer or the like. The adhesive layer of the adhesive films that constitute the puncture depth adjustment section (15) serves not only to adhere the film members (11 to 14) in the puncture depth adjustment section (15), but also to fix the administration device (18) to the skin when the administration device (18) of the present invention is punctured to the skin.

Moreover, in the present invention, both the film and the sheet refer to a web-shaped product. In the present invention, the film encompasses the meaning of sheet, and the sheet encompasses the meaning of film. The terms film and sheet should not be taken to express a difference in thickness or the like.

Further, the single layered film may be formed of a resin film made of polyolefin resin such as polyethylene, polypropylene, and cyclic polyolefin, polyester resin such as nylon and polyethylene terephthalate, polyvinyl chloride, polyvinylidene chloride, or polystyrene, polyurethane, polyvinyl alcohol, polyimide or the like, but is not limited to these materials.

Further, the shape of the film members (11 to 14) that constitute the puncture depth adjustment section (15) is not specifically limited. For example, as shown in Fig. 2, the shape is preferably a disk with an opening (annulus), in which an opening (19) has an opening area larger than an area of the portion on one surface of the substrate (10) in which the projections (16) are formed. According to this shape, the puncture depth adjustment section (15) can be provided across the entire outer periphery on the one surface of the substrate (10) without interfering with the projections (16) so that the puncture depth can be uniformly adjusted across the entire periphery. Further, the projections (16) can be punctured substantially vertically to the skin when the needle-shaped body (17) is punctured to the skin.

Further, the puncture depth adjustment section (15) may include tabs (11' to 14') on the outer edge of the film members (11 to 14) that constitute the puncture depth adjustment section (15) so that the film members (11 to 14) can be easily peeled off by holding the tab by hand.

Further, an adhesive is applied on the film members (11 to 14) that constitute the puncture depth adjustment section (15), and the substrate (10) and the film member (14) are fixed to each other. Alternatively, the film members (11 to 14) and the substrate (10) may be peelably bonded.

Further, the thickness and the numbers of sheets of the film members (11 to 14) that constitute the puncture depth adjustment section (15) may be determined considering the precision and the like required for adjustment of the length or the puncture depth of the projection (16).

As shown in Fig. 3, the puncture depth adjustment section (15) may be formed of a single film member. Further, the outer periphery of the needle-shaped body (17) and the outer periphery of the puncture depth adjustment section (15) may not be necessarily consistent with each other.

As shown in Fig. 4, the puncture depth adjustment section (15) may be shaped as an annulus having a circumference partially opened.

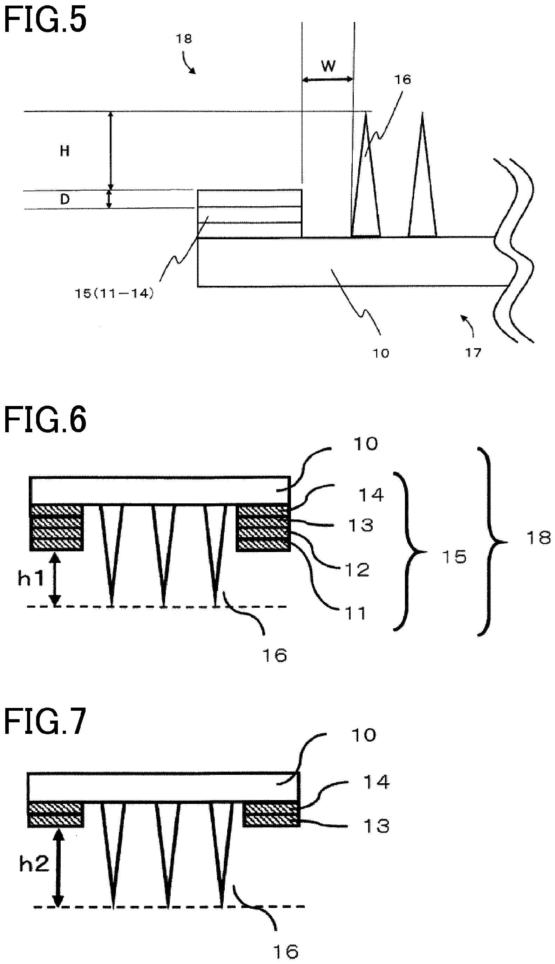

Now referring to Fig. 5, dimensions of the needle-shaped body (17) and the puncture depth adjustment section (15) will be each described. Fig. 5 is a partial cross-sectional view of the administration device (18).

A distance W from the interface between the projection (16) and the substrate (10) to the puncture depth adjustment section (15) is preferably in the range of 0.5 mm or more and 15 mm or less. When the distance W is less than 0.5 mm, it may cause difficulty in production. On the other hand, when the distance W is more than 15 mm, the puncture depth adjustment function may not be performed.

A protrusion height H of the projection (16) from the puncture depth adjustment section (15) is preferably adjusted by the puncture depth adjustment section (15) to be within the range of 50 µm or more and 3000 µm or less.

The thickness of each of the film members (11 to 14) that constitute the puncture depth adjustment section (15) is preferably in the range of 20 µm or more and 1000 µm or less.

Next, with reference to Figs. 6 and 7, an exemplary usage of the administration device (18) of the present invention will be described. Figs. 6 and 7 are cross-sectional views which illustrate a usage of the administration device (18).

First, a thickness of the skin where drug is to be administered is measured. The thickness of the skin can be measured by using a machine which uses ultrasonic waves, optical coherence tomography (OCT), or the like. The thickness of the epidermis layer and the dermis layer is measured as necessary. On the basis of the measurement result, a desired puncture depth of the projection (16) is determined. The puncture depth can also be determined depending on the ethnic group, gender, age, or puncture site.

Then, a protrusion length of the projection (16) from the puncture depth adjustment section (15) expected to be appropriate for puncturing the projection (16) at a desired depth is determined.

As shown in Fig. 6, when the thus determined protrusion length of the projection (16) from the puncture depth adjustment section (15) is the same as the initial protrusion length (h1) of the administration device (18), the film members (11 to 14) that constitute the puncture depth adjustment section (15) do not need to be peeled.

On the other hand, as shown in Fig. 7, when the thus determined protrusion length of the projection (16) from the puncture depth adjustment section (15) is larger than the initial protrusion length (h1) of the administration device (18), an appropriate number of sheets of the film members (11 to 14) that constitute the puncture depth adjustment section (15) can be peeled to obtain a desired length (h2).

Next, the administration device (18) is punctured into the skin. Puncturing of the administration device (18) into the skin is not specifically limited, and may be performed manually or by use of an applicator or the like separately provided.

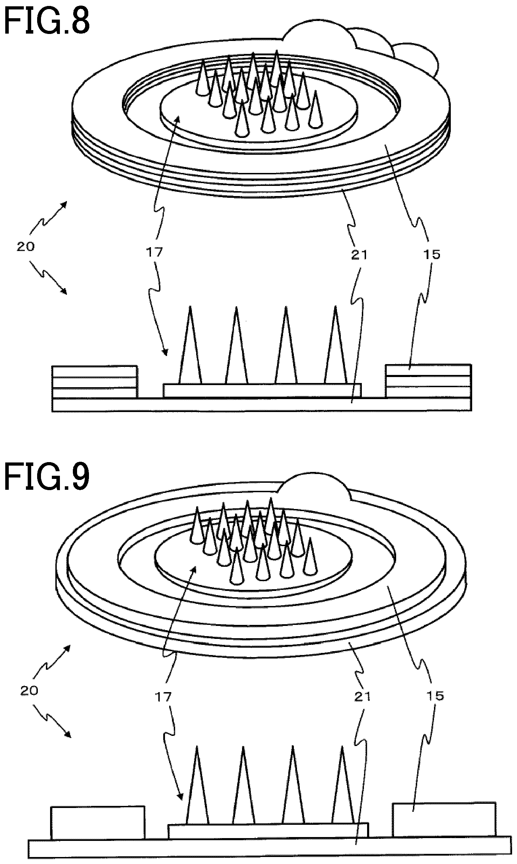

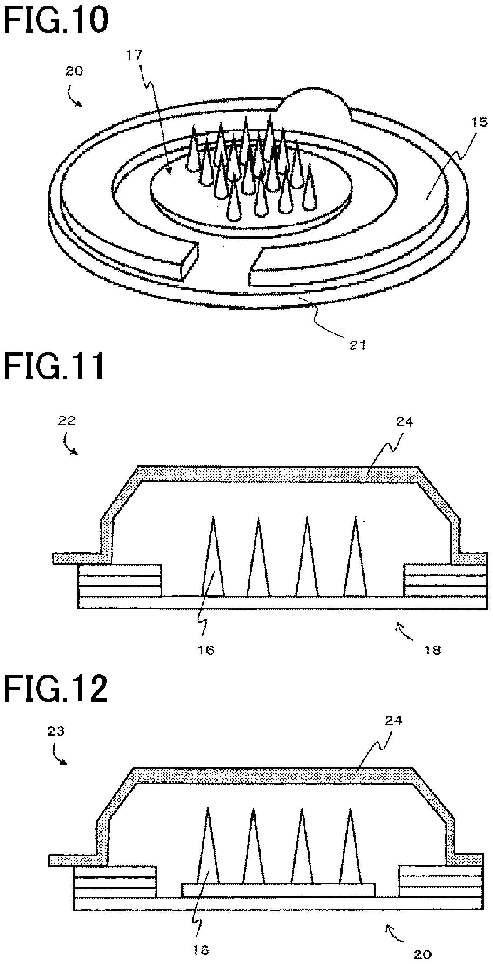

With reference to Figs. 8 to 10, an administration device (20) according to a second embodiment of the present invention will be described. Fig. 8 includes a perspective view and a cross-sectional view of the administration device (20). Fig. 9 includes a perspective view and a cross-sectional view of a first modified example of the administration device (20). Fig. 10 is a perspective view of a second modified example of the administration device (20).

As shown in Fig. 8, the administration device (20) includes a disk shaped base (21), the needle-shaped body (17) and the puncture depth adjustment section (15) which are fixed to the disk shaped base (21). The base (21) can be formed of the film member that constitutes the puncture depth adjustment section (15). In the administration device (20) as well, a height of the projection (16) from a surface of the base (21) is larger than a thickness of the puncture depth adjustment section (15). Accordingly, as shown in the cross-sectional view of Fig. 8, as viewed from the lateral side of the administration device (18) (in a horizontal direction to the drawing sheet of Fig. 8), the tip of the projection (16) protrudes from the planar surface of the puncture depth adjustment section (15).

As shown in Fig. 9, in the administration device (20), the puncture depth adjustment section (15) may be formed of a single film member. Further, the outer periphery of the base (21) and the outer periphery of the puncture depth adjustment section (15) may not be necessarily consistent with each other.

As shown in Fig. 10, in the administration device (20), the puncture depth adjustment section (15) may be shaped as an annulus having a circumference partially opened.

With reference to Figs. 11 and 12, an administration device (22, 23) with a protective cover according to a third embodiment of the present invention will be described. Figs. 11 and 12 are cross-sectional views of the administration device (22, 23) with a protective cover.

As shown in Fig. 11, the administration device (22) includes a protective cover (24) disposed on the puncture depth adjustment section (15) of the administration device (18) so as to cover the administration device (18). Further, the administration device (23) also includes the protective cover (24) disposed on the puncture depth adjustment section (15) of the administration device (20) so as to cover the administration device (20). The protective cover (24) can be attached to the puncture depth adjustment section (15) by using an adhesive or the like. In use of the administration device (18, 20), the protective cover (24) is removed.

The protective cover (24) can be made of, for example, a synthetic resin or the like. By virtue of the protective cover (24), the administration device (22, 23) can prevent damage to the projection (16) during storage and distribution processes.

### Examples

With reference to Figs. 1 and 2, examples of the administration device of the present invention will be described.

First, the administration device (18) which included the needle-shaped body (17) made of polycarbonate, and the puncture depth adjustment section (15) on which the film members (11 to 14) shaped as an annulus made of polypropylene were laminated was fabricated. The needle-shaped body (17) included the circular substrate (10) of a 20 mm diameter, and 100 projections (16) of 800 µm length arranged on the substrate (10) at a pitch of 500 µm. The film members (11 to 14), each of which had an outer diameter of cp20 mm, included the opening (19) with an inner diameter of 10 mm.

In this example, the needle-shaped body (17) was produced by injection molding. Further, the thickness of the puncture depth adjustment section (15) was 400 µm, and four film members (adhesive films) (11 to 14) with the thickness of 100 µm were laminated.

Accordingly, the administration device (18) of the present invention in which the projection (16) has the protrusion length (h1) of 400 µm from the puncture depth adjustment section (15) was obtained.

In this administration device (18), the film members (adhesive films) 11, 12, 13, and 14 in the unpeeled state were peeled off to thereby adjust the puncture depth of the projection (16) from 400 µm to approximately 500 µm, 600 µm, 700 µm, and 800 µm.

As described above, according to the present invention, the administration device (18, 20) has a simple configuration made up of the needle-shaped body (17) having the projections (16), and the puncture depth adjustment section (15). Therefore, the manufacturing cost can be reduced. Further, the puncture depth can be adjusted by simply peeling the required number of sheets of the film members (11 to 14). Therefore, ease of use can be ensured. When the needle-shaped body (17) have the projections (16) of the identical length, the puncture depth can be changed depending on the skin thickness. Accordingly, this is advantageous in terms of the production.

### [Industrial Applicability]

The present invention can be applied not only to medical field, cosmetic field, and beauty products field, but also various fields that require micropuncture techniques. For example, the present invention is useful for the fields of micro electro mechanical systems (MEMS) devices, optical devices, drug development, test drugs, nutrition supplements, beauty products, and the like.

### [Reference Signs List]

- 10: Substrate
- 11, 12, 13, 14: Film member
- 11', 12', 13', 14': Tab
- 15: Puncture depth adjustment section
- 16: Projection
- 17: Needle-shaped body
- 18, 20: Administration device
- 19: Opening
- 21: Base
- 22, 23: Administration device with a protective cover
- 24: Protective cover

## Claims

1. An administration device (18, 20) comprising:
a needle-shaped body (17) having a substrate (10) and a projection (16) protruding from one surface of the substrate (10); and
a puncture depth adjustment section (15) disposed on one surface of the substrate (10) and having at least one film member (11, 12, 13, 14) that is detachable from the needle-shaped body (17).

2. The administration device (18, 20) according to claim 1, further comprising a base (21) to which the needle-shaped body (17) and the puncture depth adjustment section (15) are fixed.

3. The administration device (18, 20) according to claim 1, wherein a height of the projection (16) is larger than a thickness of the puncture depth adjustment section (15).

4. The administration device (18, 20) according to any one of claims 1 to 3, wherein the film member (11, 12, 13, 14) is an adhesive film.

5. The administration device (18, 20) according to any one of claims 1 to 4, wherein the film member (11, 12, 13, 14) has a tab (11', 12', 13', 14').

6. The administration device (18, 20) according to any one of claims 1 to 5, wherein the film member (11, 12, 13, 14) is shaped as an annulus having an opening (19) whose opening area is larger than an area of a portion on the one surface of the substrate (10) in which the projection (16) is formed.

## Patentansprüche

1. Verabreichungsvorrichtung (18, 20), mit:
einem nadelförmigen Körper (17), der ein Substrat (10) und einen Vorsprung (16) hat, der von einer Oberfläche des Substrats (10) vorsteht; und
einem Einstichtiefeneinstellbereich (15), der an einer Oberfläche des Substrats (10) angeordnet ist und mindestens ein Folienelement (11, 12, 13, 14) hat, das von dem nadelförmigen Körper (17) abnehmbar ist.

2. Verabreichungsvorrichtung (18, 20) nach Anspruch 1, ferner mit einer Basis (21), an welcher der nadelförmige Körper (17) und der Einstichtiefeneinstellbereich (15) befestigt sind.

3. Verabreichungsvorrichtung (18, 20) nach Anspruch 1, wobei eine Höhe des Vorsprungs (16) größer ist als eine Dicke des Einstichtiefeneinstellbereichs (15).

4. Verabreichungsvorrichtung (18, 20) nach einem der Ansprüche 1 bis 3, wobei das Folienelement (11, 12, 13, 14) eine Klebefolie ist.

5. Verabreichungsvorrichtung (18, 20) nach einem der Ansprüche 1 bis 4, wobei das Folienelement (11, 12, 13, 14) eine Lasche (11', 12', 13', 14') hat.

6. Verabreichungsvorrichtung (18, 20) nach einem der Ansprüche 1 bis 5, wobei das Folienelement (11, 12, 13, 14) wie ein Kreisring geformt ist, der eine Öffnung (19) hat, deren Öffnungsfläche größer ist als eine Fläche eines Abschnitts auf der einen Oberfläche des Substrats (10), in welchem der Vorsprung (16) ausgebildet ist.

## Revendications

1. Dispositif d'administration (18, 20) comprenant :
un corps en forme d'aiguille (17) ayant un substrat (10) et une projection (16) faisant saillie depuis une surface du substrat (10) ; et
une section de réglage de profondeur de perforation (15) disposée sur une surface du substrat (10) et ayant au moins un élément film (11, 12, 13, 14) qui est détachable du corps en forme d'aiguille (17).

2. Dispositif d'administration (18, 20) selon la revendication 1, comprenant en outre une base (21) à laquelle le corps en forme d'aiguille (17) et la section de réglage de profondeur de perforation (15) sont fixés.

3. Dispositif d'administration (18, 20) selon la revendication 1, dans lequel une hauteur de la projection (16) est plus grande qu'une épaisseur de la section de réglage de profondeur de perforation (15).

4. Dispositif d'administration (18, 20) selon l'une quelconque des revendications 1 à 3, dans lequel l'élément film (11, 12, 13, 14) est un film adhésif.

5. Dispositif d'administration (18, 20) selon l'une quelconque des revendications 1 à 4, dans lequel l'élément film (11, 12, 13, 14) comporte une languette (11', 12', 13', 14').

6. Dispositif d'administration (18, 20) selon l'une quelconque des revendications 1 à 5, dans lequel l'élément film (11, 12, 13, 14) est en forme d'anneau ayant une ouverture (19) dont la superficie d'ouverture est supérieure à une superficie d'une partie sur la surface du substrat (10) dans laquelle la projection (16) est formée.
